**Europäisches Patentamt**

**European Patent Office**

**Office européen des brevets**

(19)

(11) Veröffentlichungsnummer: **0 146 740**
**B1**

(12) # EUROPÄISCHE PATENTSCHRIFT

(45) Veröffentlichungstag der Patentschrift:
**12.04.89**

(21) Anmeldenummer: **84113312.7**

(22) Anmeldetag: **06.11.84**

(51) Int. Cl.⁴: **C 08 J 9/24,** A 61 K 9/18,
A 61 K 9/20, C 08 J 9/00

(54) **Formkörper mit poröser Struktur.**

(30) Priorität: **14.11.83 US 551446**

(43) Veröffentlichungstag der Anmeldung:
**03.07.85 Patentblatt 85/27**

(45) Bekanntmachung des Hinweises auf die Patenterteilung:
**12.04.89 Patentblatt 89/15**

(84) Benannte Vertragsstaaten:
**DE FR GB**

(56) Entgegenhaltungen:
**EP-A- 0 044 051**

**CHEMICAL ABSTRACTS, Band 88, Nr. 6, 1978, Seite 41,
Zusammenfassung Nr. 38711n, Columbus, Ohio, US; &
JP - A - 77 96 679 (SEKISUI KASEIHIN KOGYO K.K.)
13-08-1977**

(73) Patentinhaber: **Akzo Patente GmbH,
Postfach 10 01 49 Kasinostrasse 19-23,
D-5600 Wuppertal-1 (DE)**

(72) Erfinder: **Castro, Anthony, 710 Roscoe, Chicago
Illinois 60657 (US)**
Erfinder: **Frank, Dieter, 25 W. 710 75th Street, Naperville
Illinois 60540 (US)**
Erfinder: **Madlock, Cleve, 821 South Williams, Westmont
Illinois 60559 (US)**

## Beschreibung

Die Erfindung betrifft Formkörper mit einer porösen Struktur, Verfahren zu deren Herstellung sowie deren Verwendung als Träger für Wirkstoffe, wie Pharmazeutika, Medikamente und dergleichen.

Es existieren zwar bereits zahlreiche Verfahren zur Herstellung poröser Körper; jedoch ist es nur mit wenigen Verfahren möglich, mikroporöse Körper herzustellen, die eine im wesentlichen dreidimensionale Ausdehnung besitzen. Ein Verfahren, mit dem man solche Körper herstellen kann, wird in der US-PS 4 247 498 beschrieben. Typische Verfahren zur Herstellung mikroporöser Materialien wie zum Beispiel das klassische Phaseninversionsverfahren eignen sich am besten zur Herstellung von Körpern wie Flachfolien. Mit Sinterverfahren lassen sich dreidimensionale mikroporöse Formkörper herstellen, die jedoch normalerweise eine hohe Dichte besitzen und ein niedriges Hohlraumvolumen aufweisen.

Mit dem Verfahren gemäs der US-PS 4 247 498 lassen sich zwar dreidimensionale Formkörper unterschiedlicher Größe herstellen; jedoch hat dieses Verfahren den Nachteil, daß die Bildung derartiger Körper aufgrund der niedrigen Extraktionsgeschwindigkeit der verträglichen Flüssigkeit einen beachtlichen Zeitaufwand erfordern kann.

In der JP-A-77/96679 (s. Chem. Abstr. 88, 38711 n) werden Filter aus Polystyrolschaum beschrieben. Zur Herstellung dieser Filter wird Polystyrolschaum pulverisiert, gesiebt und dann unter Hitzeeinwirkung bei 190 Grad C zu einem Filter geformt.

Dieses Filter hat eine verhältnismäßig hohe Durchlässigkeit für Wasser. Das gepreßte Material ist nicht geeignet mit Wirkstoffen beladen zu werden und diese langsam wieder an die Umgebung abzugeben. Dies liegt darin begründet, daß die Poren bei Schaumstoffen zum größten Teil geschlossen sind und Schaumstoffe nicht die feine mikroporöse Struktur besitzen, wie die Teilchen, welche gemäß der Erfindung zum Einsatz gelangen.

Aufgabe der Erfindung ist es, mikroporöse Formkörper zur Verfügung zu stellen, die mit Stoffen wie Wirkstoffen, z.B. Medikamenten, pharmazeutisch wirksamen Substanzen oder Medikamenten beladen werden können und die in der Lage sind, diese Stoffe in kontrollierter Weise wieder an ihre Umgebung abzugeben.

Diese Aufgabe wird durch ein Verfahren gemäß Patentanspruch 1 gelöst.

Besonders vorteilhafte Ausführungsformen dieses erfindungsgemäßen Verfahrens werden in den Patentansprüchen 2 bis 13 beschrieben.

Gegenstand der Erfindung sind ferner die Verwendung der Formkörper gemäß Patentanspruch 14 sowie Formkörper gemäß Patentanspruch 15.

Im folgenden wird die Erfindung anhand der Figuren 1 bis 6 näher erläutert.

Fig. 1 ist eine graphische Darstellung der Dichte von erfindungsgemäßen Körpern aus mikroporösem Polypropylen in Abhängigkeit des bei der Herstellung der Körper angewandten Druckes.

Fig. 2 ist eine graphische Darstellung des Hohlraumvolumenanteils von Körpern gemäß der Erfindung, die aus drei verschiedenen mikroporösen Polypropylen-Pulvern hergestellt worden sind und die ein Hohlraumvolumen von 60, 70 und 80% aufweisen, in Abhängigkeit des bei der Herstellung der Körper angewandten Druckes.

Fig. 3 ist eine graphische Darstellung der Biegefestigkeit von erfindungsgemäßen Körpern, die aus mikroporösem Polypropylenpulver hergestellt worden sind und die Hohlraumvolumen von 60, 70 bzw. 80% aufweisen, in Abhängigkeit von der scheinbaren Porosität der Körper.

Fig. 4 ist eine graphische Darstellung, in der die Biegefestigkeit von Körpern gemäß der vorliegenden Erfindung, hergestellt aus mikroporösem Polyamid 6 und solcher aus Hochdruckpolyäthylen, miteinander verglichen werden.

Fig. 5 ist eine rasterelektronenmikroskopische Aufnahme (in 1050-facher Vergrößerung) eines Frostbruches eines erfindungsgemäßen Produktes, die zeigt, daß im wesentlichen kein Zwischenhohlraum mehr vorhanden ist.

Fig. 6 ist eine rasterelektronenmikroskopische Aufnahme (in 1050-facher Vergrößerung) eines Körpers, hergestellt aus nichtporösen Teilchen aus festem Polypropylen, die die Gegenwart von wesentlichen Zwischenhohlräumen und eine Abflachung der ursprünglich kugelförmigen Teilchen zeigt.

Wie bereits aufgezeigt, stellt das vorliegende Verfahren eine einfache Methode zur Herstellung von dreidimensionalen porösen Körpern dar. Mit Hilfe der vorliegenden Erfindung ist es möglich, poröse Körper mit einer Dichte herzustellen, die niedriger ist als die theoretische Dichte eines gleichen Körpers, der lediglich aus nichtporösen Teilchen des gleichen Materials unter gleichem Druck hergestellt worden ist.

Die Ausgangsstoffe sind Teilchen eines synthetischen polymeren Materials. Das Material kann ein Homopolymer, ein Copolymer oder eine Mischung aus verschiedenen Homopolymeren und/ oder Copolymeren sein. Das Verfahren gemäß der vorliegenden Erfindung ist anwendbar auf olefinische Polymere, Kondensationspolymere und Oxidationspolymere. Besonders geeignet als synthetisches polymeres Material sind hochpolymeres Polypropylen oder Polyäthylen.

Beispiele für brauchbare nichtacrylische Polyolefine sind
Niederdruck-Polyäthylen, Hochdruck-Polyäthylen, Polypropylen, Polystyrol, Polyvinylchlorid, Acrylnitril-Butadien-Styrol-Terpolymere, Styrol-Acrylnitril-Copolymere, Styrol-Butadien-Copolymere, Poly(4-Methyl-Penten-1), Polybutylen, Polyvinylidenchlorid, Polyvinylburyral, chloriertes Polyäthylen, Äthylenvinylacetat-Copolymere, Polyvinylacetat und Polyvinylalkohol.

Geeignete Acrylpolyolefine sind Polymethyl-Methacrylat, Polymethyl-Acrylat, Äthylen-Acryl-

säure-Copolymere und Äthylen-Acrylsäure-Metallsalze-Copolymere.

Polyphenylenoxid ist ein sehr geeignetes Oxidationspolymer. Geeignete Kondensationspolymere sind Polyäthylenterephthalat, Polybutylenterephthalat, Polyamid 6, Polyamid 11, Polyamid 13, Polyamid 6.6, Polycarbonate und Polysulfon. Die in einer Ausführungsform der vorliegenden Erfindung zu verwendenden nichtporösen Teilchen lassen sich durch einfaches Mahlen und Sieben von festem Kunststoff beliebiger Form herstellen. Derartige Teilchen sind außerdem im Handel erhältlich.

Die mikroporösen Teilchen lassen sich zwar mit allen Verfahren herstellen, die derartige Teilchen aus dem gewünschten synthetischen polymeren Material ergeben; bevorzugt wird jedoch nach einem Verfahren gemäß der US-PS 4 247 498 gearbeitet. Mit diesem Verfahren kann man zunächst einen Ausgangskörper beliebiger Form herstellen, vorzugsweise in einer Form, die leicht zugänglich und leicht mahlbar ist. Dieser Ausgangskörper kann dann cryogen gemahlen werden, wobei man eine käuflich erhältliche Vorrichtung für diesen Zweck einsetzen kann; sodann werden die Teilchen extrahiert. Die Teilchen, welche man von diesem Körper erhält, können dann durch Sieben nach ihrer Teilchengröße sortiert werden. Es werden vorzugsweise die Teilchen verwendet, die in einem Größenordnungsbereich von etwa 40 bis etwa 400 µm liegen.

Weitere Verfahren zur Herstellung von mikroporösen Teilchen, die vorteilhaft im Rahmen der Erfindung verwendet werden können, sind in den US-PS 4 391 920, 4 379 860 und 4 454 198 beschrieben, welche mit den DE-OS 3 026 688 und 3 026 762 korrespondieren. Gemäß der vorliegenden Erfindung müssen mindestens 5% der eingesetzten Teilchen mikroporös sein und besitzen vorzugsweise ein hohes Hohlraumvolumen, z.B. etwa 50 bis etwa 90%, vorzugsweise etwa 70 bis etwa 80%. Die Größe der Mikroporen ist nicht kritisch und liegt normalerweise im Bereich von etwa 0,05 µm bis etwa 5 µm, vorzugsweise von etwa 0,2 bis etwa 1,0 µm.

Wie bereits erwähnt, können gemäß der Erfindung Körper von praktisch jeder beliebigen Gestalt hergestellt werden. Insbesondere können jedoch Tabletten oder Scheiben geformt werden, wobei einfache Vorrichtungen verwendet werden, um die Teilchen in der gewünschten Form zu halten. Auf diese Teilchen wird ein äußerer Druck angewandt, z.B. unter Verwendung einer hydraulischen Presse, wie sie zum Beispiel verwendet wird, wenn man KBr-Tabletten für die Infrarot-Spektroskopie herstellt. Es können weiter Vorrichtungen zum Pressen von Tabletten eingesetzt werden, wie sie in der pharmazeutischen Industrie üblich sind.

Der angewandte Druck kann zwischen ca. $13,8 \cdot 10^5$ bis ca. $551 \cdot 10^5$ Pa liegen. Im allgemeinen erhält man eine beträchtliche Festigkeit, wenn der angewandte Druck ca. $20,7 \cdot 10^5$ Pa oder mehr beträgt. Wenn der Druck jedoch $107 \cdot 10^5$ Pa überschreitet und sich einem Wert von etwa $172,5 \cdot 10^5$ Pa nähert, erreicht die Dichte der erhaltenen Körper den theoretischen Wert für die Schüttdichte von nichtporösen Polypropylenkugeln in einer hexagonalen dichtgepackten Anordnung. Man könnte daher annehmen, daß die verbleibende Porosität im wesentlichen den Zwischenräumen entspricht. Aus rasterelektronenmikroskopischen Aufnahmen («SEMs») geht jedoch hervor, daß die ursprünglichen mikroporösen Teilchen zunächst abflachen, so daß die Zwischenräume beseitigt werden, bevor die Mikroporen zu verschwinden beginnen.

So kann zum Beispiel die verbleibende Porosität einer bei $145 \cdot 10^5$ Pa hergestellten Probe 33% betragen, wovon noch ein wesentlicher Teil immer noch Mikroporen sind und nicht ein Zwischenraum zwischen den kugelförmigen Teilchen, der theoretisch 31% in einer hexagonalen dichtgepackten Anordnung beträgt.

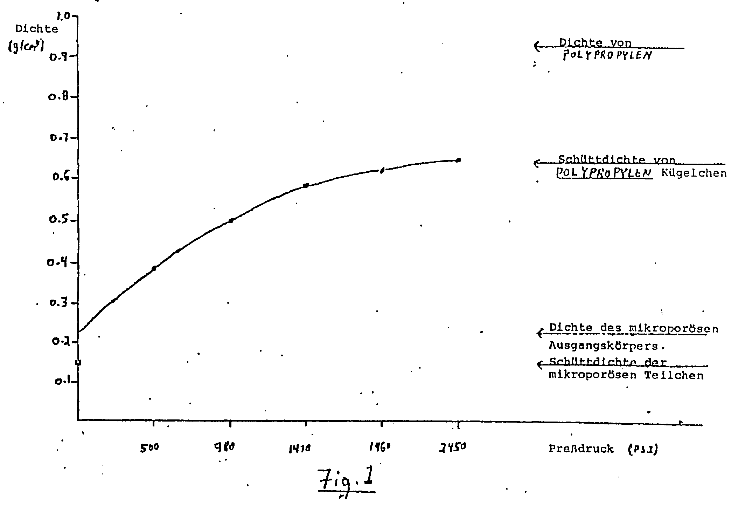

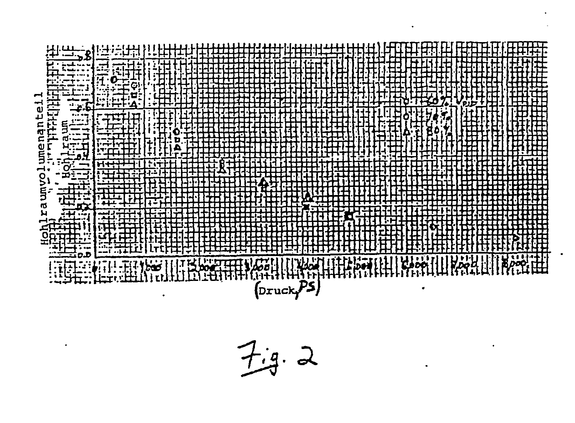

Es ist überraschend, daß sich aus den relativ zerbrechlichen mikroporösen Teilchen gemäß der vorliegenden Erfindung ganze Körper formen lassen, die einen im wesentlichen physikalischen Zusammenhalt aufweisen, der fest genug ist, daß sich die Körper handhaben lassen, und daß diese Körper nach wie vor einen Teil der ursprünglich vorhandenen Mikroporosität besitzen. Es ist besonders überraschend, daß gemäß der Erfindung relativ feste kompakte Verbundträger zugänglich sind, ohne daß hierfür äußere Hitzeeinwirkung, Lösungsmittel, Binder, oder sonstige Mittel erforderlich wären, um die Teilchen an ihren Rändern miteinander zu verbinden. Es ist ferner überraschend, daß bei Anwendung von Druck auf Ausgangsteilchen mit verschiedenem Porositätsgrad wie 60 und 80% das entstehende Hohlraumvolumen in den Formkörpern praktisch nur von der Höhe des angewendeten Drucks abhängig ist (Fig. 2).

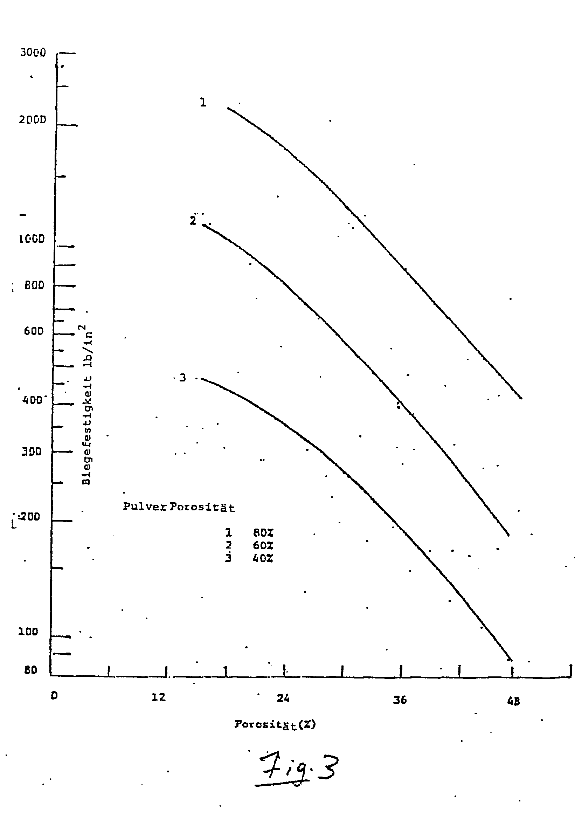

Die mechanische Festigkeit der Formkörper ist allerdings in starkem Maße von der Ausgangsporosität abhängig. So ergeben Teilchen mit 80% Ausgangsporosität Tabletten mit einer doppelt so hohen Festigkeit wie bei der Verwendung von Teilchen mit einer Ausgangsporosität von 60% (Fig. 3). Daraus ergibt sich, daß es den Anschein hat, daß eine Erhöhung der Ausgangsmikroporosität zu einer Erhöhung der Bindekräfte und somit zu einer erhöhten Festigkeit des gepreßten Formkörpers führt.

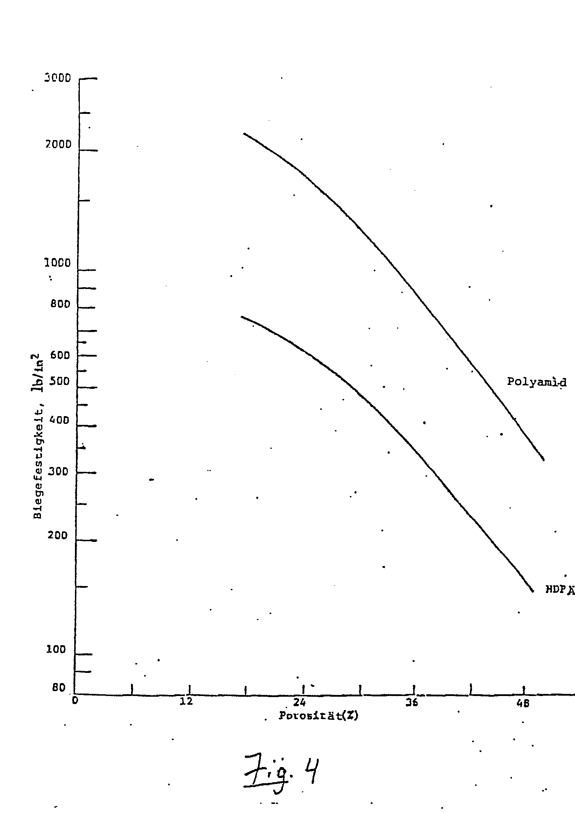

Zu erwarten wäre auch, daß aufgrund der unterschiedlichen Oberflächenkräfte Teilchen, die aus verschiedenen Polymeren hergestellt worden sind, bei Anwendung gleicher Drucke Körper unterschiedlicher Festigkeit ergäben, was für Polypropylen, Polyamid und Hochdruck-Polyäthylen, deren Festigkeit in dieser Reihenfolge abnimmt, auch tatsächlich zutrifft (Fig. 3 und 4).

Gegenstand der Erfindung sind ferner Formkörper, die ganz oder teilweise mit einem Wirkstoff wie pharmazeutisch wirksamen Substanzen oder Medikamenten beladen sind. So können die mikroporösen Teilchen zumindest teilweise mit einem Wirkstoff wie einem Medikament beladen werden. Diese so beladenen Teilchen, die an sich noch die ursprüngliche Mikroporosität aufweisen, können zur Herstellung der gewünschten Formkörper verwendet werden. So kann man z.B. die Teilchen schnell beladen, indem man sie in eine Wirkstofflösung eintaucht und anschließend das Lösungsmittel durch Verdampfen entfernt. Danach werden die beladenen Teilchen zu einem zusammenhängenden Formkörper gepreßt, der den gewünschten Wirkstoff enthält. Auf diese Weise kann man die Notwendigkeit umgehen, den wesentlich größeren Formkörper in eine solche Lösung einzutauchen, wobei man, um die erforderliche Durchdringung zu erreichen, wesentlich längere Zeiträume benötigt, und dann den Formkörper einem längeren Trocknungsprozeß zu unterziehen, um das Lösungsmittel zu verdampfen. In einer anderen Verfahrensweise können dem mikroporösen Polymer Farbpigmente während des Verfahrens zugesetzt werden, das in der US-PS 4 247 498 beschrieben wird und auf diese Weise in die Formkörper eingelagert werden.

Selbstverständlich können die mikroporösen Teilchen auch noch auf andere Weise mit Wirkstoffen versehen werden.

Wie bereits ausgeführt, scheinen die physikalischen Kräfte, welche die mechanische Festigkeit in dem Preßkörper bewirken, besonderer Art zu sein und sich aus der besonderen mikroporösen Struktur zu ergeben.

Das Beladen der genannten Körper mit gewissen Wirkstoffen vor dem Pressen führt nicht zu einer wesentlichen Verringerung der in dem Verfahren genutzten oberflächenwirksamen Bindekräfte.

Die Erfindung wird anhand der folgenden Beispiele näher erläutert:

Beispiel 1

Nach dem Verfahren gemäß US-PS 4 247 498 wurde ein mikroporöser Festkörper aus 25 Gew.-% Polypropylen und 75 Gew.-% Armostat(R) 310 N,N-bis(2-Hydroxyäthyl)Talgamin hergestellt. Die dabei erhaltene Struktur wurde unter Anwendung von Kälte zu mikroporösen Teilchen zermahlen und danach mit Isopropanol zwecks Entfernung des Amins extrahiert, getrocknet und zur Entfernung der Feinteilchen gesiebt, so daß die erhaltenen Teilchen eine Größe von ca. 40 bis ca. 400 µm aufwiesen. Solche Teilchen sind als mikroporöses Pulver unter dem Warenzeichen Accurel(R) von der Firma Armak Company, Chicago, Illinois, erhältlich.

Das genannte Pulver wurde in einer hydraulischen Presse bei einem effektiven Druck von ca. 34,5 – ca. $169 \cdot 10^5$ Pa zu Tabletten mit einem Durchmesser von 3,175 cm und einer Dicke zwischen 4 und 8 mm geformt. Abbildung 1 zeigt die Dichte der erhaltenen Tabletten im Verhältnis zum Preßdruck in psi. Alle Tabletten wiesen eine gute mechanische Festigkeit auf und ließen sich leicht handhaben, ohne daß es dabei zu einem Abbau des Produktes kam.

Die Dichte des ursprünglichen mikroporösen Festkörpers, aus dem die mikroporösen Teilchen hergestellt wurden, betrug ca. 0,225 g/cm³ und die Schüttdichte des daraus hergestellten Pulvers betrug 0,147 g/cm³.

Es ist bekannt, daß sich Kugeln am dichtesten packen lassen, wenn man sie hexagonal anordnet, und daß bei einer solchen Anordnung die Kugeln 69% des Gesamtvolumens einnehmen. Somit wäre zu erwarten, daß bei gleichmäßigen mikroporösen Teilchen in einer hexagonalen Anordnung die Dichte des Pulvers 69% der Dichte der Teilchen selbst (0,225 g/cm³) bzw. 0,155 g/cm³ entspräche. Somit lag die gemessene Dichte nur um ca. 5% unter der für eine hexagonale Anordnung errechneten theoretischen Dichte.

Bei den verschiedenen angewandten Drücken wiesen die erhaltenen Strukturen folgende Dichte auf:

| Angewandter Druck in Pa | Gemessene Dichte g/cm³ |
|---|---|
| $34,5 \cdot 10^5$ | 0,3839 |
| $67,5 \cdot 10^5$ | 0,4953 |
| $101,5 \cdot 10^5$ | 0,5808 |
| $135 \cdot 10^5$ | 0,6121 |
| $169 \cdot 10^5$ | 0,6408 |

Wie bereits oben erwähnt, wird angenommen, daß die Porosität der erhaltenen Struktur bei Druckkräften unter ca. 169 Pa hauptsächlich auf die ursprüngliche Mikroporosität der Teilchen und weniger auf die Zwischenräume zwischen den Kugeln zurückzuführen ist.

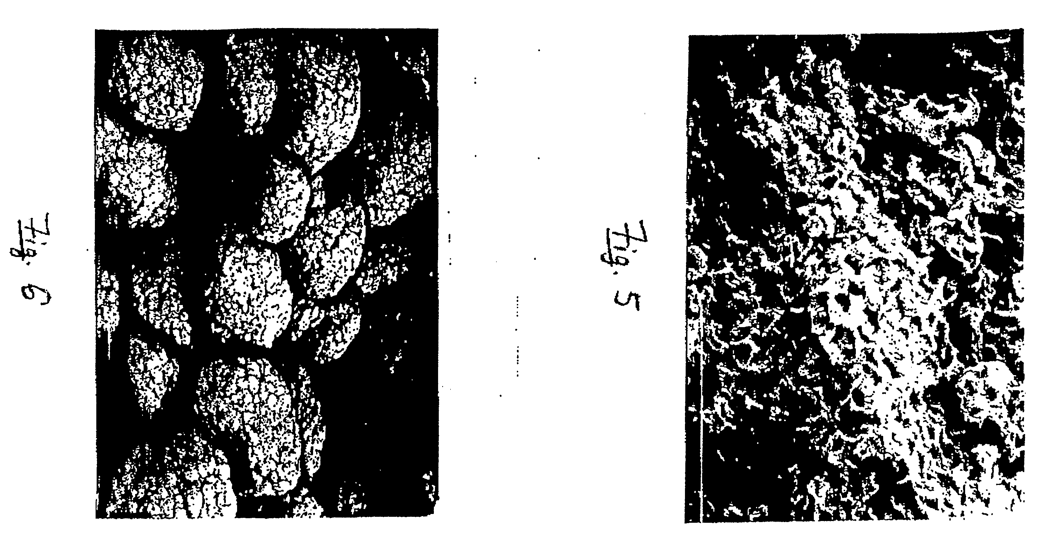

Auf Abbildung 5 ist eine rasterelektronenmikroskopische Aufnahme (in 1050-facher Vergrößerung) eines Frostbruches eines bei einem Druck von ca. $114 \cdot 10^5$ Pa erhaltenen Produktes dargestellt, aus der hervorgeht, daß im wesentlichen keinerlei Zwischenräume mehr vorhanden sind.

Beispiel 2 (Vergleich)

Um die reine Anwendung von Druck auf nur aus festem Polypropylen bestehenden Teilchen zu demonstrieren, wurden derartige vergleichbare Teilchen in derselben hydraulischen Presse verschiedenen Drücken ausgesetzt. Unter ca. $169 \cdot 10^5$ Pa konnten keine zusammenhaltenden Tabletten hergestellt werden; die Ränder waren weich, und es kam zu einem Abrieb von Pulverteilchen. Bei Drücken von ca. 169 – ca. $676 \cdot 10^5$ Pa wiesen die erhaltenen Strukturen mit den erfindungsgemäß bei Drücken von ca. 17,25 – ca. $34,5 \cdot 10^5$ Pa hergestellten Tabletten vergleichbare Festigkeiten und Ränder auf. Die Dichte der aus den festen Polypropylenteilchen hergestellten Strukturen betrug in allen Fällen annähernd 0,7 g/cm³.

Polypropylen besitzt eine Dichte von 0,92 g/cm³, so daß eine theoretische Struktur aus gleichmäßigen Kugeln aus festem Polypropylen eine Dichte von 0,92 g/cm³ × 0,69 bzw. 0,63 g/cm³ aufweisen dürfte. Die geringe Erhöhung der Dichte der komprimierten Körper ist auf die Abflachung der ursprünglich kugelförmigen Oberflächen zurückzuführen, wie aus den rasterelektronenmikroskopischen Aufnahmen hervorgeht. Abbildung 6 zeigt eine rasterelektronenmikroskopische Aufnahme (in 1050-facher Vergrößerung) eines Frostbruches einer bei einem Druck von $562,5 \cdot 10^5$ Pa hergestellten Struktur, auf der eine beträchtliche Anzahl von Zwischenräumen sowie die Abflachung der ursprünglich kugelförmigen Oberflächen zu sehen sind. Eine ausreichende Festigkeit läßt sich erst nach Abflachung der kugelförmigen festen Teilchen erreichen, da nur auf diese Weise ein ausreichender Oberflächenkontakt mit den entsprechenden Kohäsionskräften zustande kommt. Die Festigkeit derartiger Produkte liegt jedoch noch unter dem bei Einsatz von mikroporösem Material bei gleichem Druck erzielten Niveau.

Beispiel 3

Ein gemäß US-Patentschrift 4 247 498 hergestelltes mikroporöses Polypropylenpulver mit einem Porenvolumen von 75% wurde mit Koffein durch Lagerung des Pulvers in einer 8% gelöstes Koffein enthaltenden Äthanol-Lösung (60/40) bei 55 °C beladen. Die Entfernung des Lösungsmittels erfolgte durch Trocknen im Vakuum bei Trockeneistemperatur. Das 30 Gew.-% Koffein enthaltende Trockenpulver wurde mit einem Druck von $345 \cdot 10^5$ Pa, wie in Beispiel 2 beschrieben, zu einer Tablette verpreßt. Die Tablette wies eine gute mechanische Festigkeit auf. Der etwas höhere Preßdruck eignet sich gut zur Erzielung des obengenannten Verhältnisses zwischen mechanischer Festigkeit und Porenvolumen (gemäß Abbildung 3). In analoger Weise erfolgte eine Beladung von Pulver mit dem Farbstoff Orasol Orange mit nachfolgendem Verpressen zu einer Scheibe.

Beispiel 4

Eine Mischung aus 700 g Armostat(R) 310 Amin, 50 g Inmont Red 17-73238 Liqui-Kolor(R) (eine Dispersion von rotem Farbstoff in einem Öl-Medium mit einem Feststoffgehalt von 50%) und 250 g Polypropylen wurden auf 200 °C bis zur Erreichung einer homogenen Mischung erhitzt. Die Mischung wurde dann zu einer ¼ dicken Platte gegossen und auf Raumtemperatur abkühlen gelassen. Die Platte wurde granuliert, gemahlen und mit Aceton extrahiert, wobei ein poröses Polymerpulver mit einem Farbstoffgehalt von ca. 9% entstand. Das Pulver wurde bei $172,5 \cdot 10^5$ Pa in einer Form mit einem Durchmesser von 3,185 cm nach Verfahren gemäß Beispiel 1 zu Tabletten mit einer mit Tabletten aus nicht-beladenem mikroporösen Polypropylen vergleichbaren Festigkeit verdichtet.

Beispiel 5

6 Gew.-% mikroporöses nach US-Patentschrift 4 247 498 hergestelltes Polypropylen wurde mit 94% festem Polypropylenpulver gemischt und bei $138 \cdot 10^5$ Pa gepreßt. Die dabei entstandene Tablette wies eine ausgezeichnete mechanische Festigkeit und keinen Abrieb von Teilchen wie im Falle von Beispiel 2 auf.

Beispiel 6

Mikroporöse gemäß US-Patentschrift 4 247 498 unter Verwendung von Armostat(R) 310 Amin als

geeignete Flüssigkeit hergestellte Hochdruck-Polyäthylen-Teilchen mit einem Porenvolumen von 75% wurden nach dem Gefriermahlen mit folgender Extraktion in einer hydraulischen Presse zu Tabletten gepreßt. In gleicher Weise wurden gemäß US-Patentschrift 4 247 498 unter Verwendung von Propylencarbonat als geeignete Flüssigkeit hergestellte mikroporöse Polyamid-6-Teilchen mit einem Porenvolumen von 80% nach dem Gefriermahlen in einer hydraulischen Presse zu Tabletten gepreßt. Abbildung 4 zeigt die Biegefestigkeit der erhaltenen Strukturen im Verhältnis zu ihrer Porosität. Strukturen aus Polyamid 6 besitzen, wie erwartet, eine höhere Biegefestigkeit als Strukturen aus Hochdruck-Polyäthylen, jedoch eine geringere Biegefestigkeit als mit der gleichen Druckkraft hergestellte Strukturen aus Polypropylen.

**Patentansprüche**

1. Verfahren zur Herstellung von mikroporösen Formkörpern durch Formen einer Vielzahl von Teilchen zu der gewünschten Gestalt ohne äußere Hitzeeinwirkung, wobei die Größe der Teilchen in einem Bereich von etwa 40 bis etwa 400 μm liegen und die Teilchen zu ca. 5 bis ca. 100 Gew.-% aus mikroporösen Teilchen eines synthetischen thermoplastischen polymeren Materials und zu ca. 0 bis ca. 95 Gew.-% aus nichtporösem synthetischem thermoplastischem polymeren Material bestehen und die Teilchen einem effektiven Druck von ungefähr $13,8 \cdot 10^5$ bis ungefähr $551 \cdot 10^5$ Pa ausgesetzt werden, während man die Teilchen in der gewünschten Form für einen Zeitraum hält, der ausreicht, um einen zusammenhängenden, porösen Formkörper zu erzeugen, der einen im wesentlichen physikalischen Zusammenhalt aufweist.

2. Verfahren nach Anspruch 1, bei dem man als synthetisches thermoplastisches Material Niederdruck-Polyäthylen, Hochdruck-Polyäthylen, Polypropylen, Polystyrol, Polyvinylchlorid, Acrylnitril-Butadien-Styrol-Terpolymere, Styrol-Acrylnitril-Copolymer, Styrol-Butadien-Copolymere, Poly-(4-Methylpenten-1), Polybutylen, Polyvinylidenchlorid, Polyvinylbutyral, chloriertes Polyäthylen, Äthylen-Vinylacetat-Copolymerisate, Polyvinylacetat und Polyvinylalkohol verwendet.

3. Verfahren nach Anspruch 1, bei dem man als synthetisches thermoplastisches Material Polymethyl-Methacrylat, Polymethyl-Acrylat, Äthylen-Acrylsäure-Copolymere oder Äthylen-Acrylsäure-Metallsalze-Copolymere verwendet.

4. Verfahren nach Anspruch 1, bei dem man als synthetisches thermoplastisches Material Polyphenylenoxyd, Polyäthylenterephthalat, Polybutylenterephthalat, Polyamid 6, Polyamid 11, Polyamid 13, Polyamid 66, Polycarbonate oder Polysulfone verwendet.

5. Verfahren nach einem der Ansprüche 1 bis 4, bei welchem die mikroporösen Teilchen ein Hohlraumvolumen von etwa 70 bis etwa 80% besitzen.

6. Verfahren nach einem der Ansprüche 1 bis 5, bei welchem die Teilchen Poren im Bereich von etwa 0,05 μm bis etwa 5 μm aufweisen.

7. Verfahren nach einem der Ansprüche 1 bis 6, bei welchem der Druck ca. 20,7 · 10⁵ bis ca. 240 · 10⁵ Pa beträgt.

8. Verfahren nach einem der Ansprüche 1 bis 7, bei welchem man die mikroporösen Teilchen zumindest teilweise mit einem Wirkstoff belädt.

9. Verfahren nach Anspruch 8, bei welchem man als Wirkstoff ein Medikament verwendet.

10. Verfahren nach Anspruch 8, bei welchem man als Wirkstoff eine pharmazeutisch wirksame Substanz verwendet.

11. Verfahren nach einem der Ansprüche 8 bis 10, bei welchem man die mikroporösen Teilchen vor dem Formen mit einem Wirkstoff belädt.

12. Verfahren nach einem der Ansprüche 1 bis 11, bei welchem man die Teilchen zu Tabletten formt.

13. Verfahren nach einem der Ansprüche 1 bis 7, bei welchem man die mikroporösen Teilchen mit Farbpigmenten belädt.

14. Verwendung der Formkörper, hergestellt nach einem Verfahren gemäß der Ansprüche 1 bis 7, als Träger von Pharmazeutika oder Medikamenten.

15. Ganz oder teilweise mit einer pharmazeutisch wirksamen Substanz oder einem Medikament beladener, nach einem Verfahren gemäß Patentanspruch 1 hergestellter Formkörper.

**Claims**

1. A process for the production of microporous mouldings by moulding a plurality of particles into the required shape without external application of heat, the size of the particles being in the range from about 40 to about 400 μm and approximately 5 to approximately 100% by weight of the particles consisting of microporous particles of a synthetic thermoplastic polymeric material and approximately 0 to approximately 95% by weight of nonporous, synthetic, thermoplastic polymeric material and the particles being exposed to an effective pressure of approximately 13.8 × 10⁵ to approximately 551 × 10⁵ Pa while the particles are held in the required shape for a period sufficient to produce a coherent, porous moulding which shows substantially physical cohesion.

2. A process as claimed in claim 1, in which the synthetic thermoplastic material used is selected from
low-pressure polyethylene,
high-pressure polyethylene, polystyrene,
polyvinyl chloride,
acrylonitrile-butadiene-styrene terpolymers,
styrene-acrylonitrile copolymer,
styrene-butadiene copolymers,
poly-(4-methyl-1-pentene), polybutylene,
polyvinylidene chloride, polyvinyl butyral,
chlorinated polyethylene,
ethylene-vinyl acetate copolymers,
polyvinyl acetate and polyvinyl alcohol.

3. A process as claimed in claim 1, in which the synthetic thermoplastic material used is selected from polymethyl methacrylate, polymethyl acrylate, ethylene-acrylic acid copolymers or ethylene-acrylic acid-metal salt copolymers.

4. A process as claimed in claim 1, in which the synthetic thermoplastic material used is polyphenylene oxide, polyethylene terephthalate, polybutylene terephthalate, polyamide 6, polyamide 11, polyamide 13, polyamide 66, a polycarbonate or a polysulfone.

5. A process as claimed in any of claims 1 to 4, in which the microporous particles have a pore space volume of about 70 to about 80%.

6. A process as claimed in any of claims 1 to 5, in which the particles have pores in the range from about 0.05 μm to about 5 μm.

7. A process as claimed in any of claims 1 to 6, in which the pressure is in the range from approximately 20.7 × 10⁵ to approximately 240 × 10⁵ Pa.

8. A process as claimed in any of claims 1 to 7, in which the microporous particles are at least partly charged with an active substance.

9. A process as claimed in claim 8, in which a medicament is used as the active substance.

10. A process as claimed in claim 8, in which a pharmaceutically active substance is used as the active substance.

11. A process as claimed in any of claims 8 to 10, in which the microporous particles are charged with an active substance before moulding.

12. A process as claimed in any of claims 1 to 11, in which the particles are moulded into tablets.

13. A process as claimed in any of claims 1 to 7, in which the microporous particles are charged with coloured pigments.

14. The use of the mouldings produced by the process claimed in claims 1 to 7 as carriers for pharmaceuticals or medicaments.

15. Mouldings produced by the process claimed in claim 1 charged completely or partly with a pharmaceutically active substance or a medicament.

**Revendications**

1. Procédé pour la fabrication de corps moulés microporeux, par moulage d'un grand nombre de particules en la forme désirée, sans apport de chaleur externe, la taille des particules étant dans une plage d'environ 40 à environ 400 μm et les particules étant constituées, à raison d'environ 5 à environ 100% en poids, de particules microporeuses d'un matériau polymère synthétique thermoplastique, et à raison d'environ 0 à environ 95% en poids, de matériau polymère synthétique thermoplastique non poreux, et les particules étant soumises à une pression effective d'environ 13,8 · 10⁵ à environ 551 · 10⁵ Pa, pendant que l'on maintient les particules dans le module désiré, pendant une durée suffisante pour la production d'un corps moulé cohérent, poreux, qui présente une cohésion essentiellement physique.

2. Procédé selon la revendication 1, dans lequel on utilise, en tant que matériau synthétique thermoplastique,
un polyéthylène basse pression,
un polyéthylène haute pression,
du polypropylène, du polystyrène,
du poly(chlorure de vinyle),
des terpolymères acrylonitrile/butadiène/styrène,
un copolymère styrène/acrylonitrile,
des copolymères styrène/butadiène,
du poly(4-méthylpentène-1), du polybutylène,
du poly(chlorure de vinylidène),
du poly(butyral de vinyle),
du polyéthylène chloré,
des copolymères éthylène/acétate de vinyle,
du poly(acétate de vinyle) ou du poly(alcool vinylique).

3. Procédé selon la revendication 1, dans lequel
on utilise, en tant que matériau synthétique thermoplastique,
du poly(méthacrylate de méthyle),
du poly(acrylate de méthyle),
des copolymères éthylène/acide acrylique
ou des copolymères éthylène/sels métalliques
d'acides acrylique.

4. Procédé selon la revendication 1, dans lequel
on utilise, en tant que matériau synthétique thermoplastique,
du polyoxyphénylène, du poly(téréphtalate
d'éthylène),
du poly(téréphtalate de butylène),
du polyamide 6, du polyamide 11,
du polyamide 13, du polyamide 66,
des polycarbonates ou des polysulfones.

5. Procédé selon l'une des revendications 1 à 4,
dans lequel les particules microporeuses ont un
volume de cavité d'environ 70 à environ 80%.

6. Procédé selon l'une des revendications 1 à 5,
dans lequel les particules présentent des pores
dans la plage d'environ 0,05 μm à environ 5 μm.

7. Procédé selon l'une des revendications 1 à 6,
dans lequel la pression est d'environ $20,7 \cdot 10^5$ à
environ $240 \cdot 10^5$ Pa.

8. Procédé selon l'une des revendications 1 à 7,
dans lequel on charge les particules microporeuses, au moins partiellement, avec une substance
active.

9. Procédé selon la revendication 8, dans lequel, en tant que substance active, on utilise un
médicament.

10. Procédé selon la revendication 8, dans lequel, en tant que substance active, on utilise une
substance à activité pharmaceutique.

11. Procédé selon l'une des revendications 8 à
10, dans lequel on charge les particules microporeuses avec une substance active, avant le moulage.

12. Procédé selon l'une des revendications 1 à
11, dans lequel on moule les particules en comprimés.

13. Procédé selon l'une des revendications 1 à
7, dans lequel on charge les particules microporeuses avec des pigments colorés.

14. Utilisation des corps moulés, fabriqués par
un procédé selon les revendications 1 à 7, en tant
que véhicule de produits pharmaceutiques ou de
médicaments.

15. Corps moulé, chargé en partie ou en totalité
d'une substance à activité pharmaceutique ou
d'un médicament, fabriqué par un procédé selon
la revendication 1.

Fig. 1

EP 0 146 740 B1

Fig. 2

Fig. 3

Fig. 4

Fig. 5

Fig. 6